# EUROPEAN PATENT APPLICATION

(11) **EP 4 578 846 A1**
(43) Date of publication of application: **02.07.2025**
(21) Application number: 23857438.8
(22) Date of filing: 25.08.2023
(51) Int. Cl.: C07C 5/25, B01J 21/04, B01J 21/20, B01J 38/02, C07B 61/00, C07C 11/02

(54) **METHOD FOR PRODUCING INTERNAL OLEFIN AND METHOD FOR REGENERATING ACTIVITY OF CATALYST**

(30) Priority: 26.08.2022 JP 2022135013
(71) Applicant: Kao Corporation, Tokyo 103-8210 (JP)
(72) Inventor: IKEBATA Hidehito, Wakayama-shi, Wakayama 640-8580 (JP); TAKADA Shingo, Wakayama-shi, Wakayama 640-8580 (JP)
(74) Representative: Hoffmann Eitle
(86) International application number: PCT/JP2023/030695
(87) International publication number: WO 2024/043330

(57) **Abstract**

A method for producing an internal olefin, including a step of subjecting a raw material olefin to an isomerization reaction in the presence of the following catalyst R: Catalyst R: a catalyst obtained by holding a catalyst, which has been subjected to an isomerization reaction of an olefin at a reaction temperature (T₁), at a temperature higher than the reaction temperature (T₁) (hereinafter also referred to as a "catalyst holding temperature (T₂)") in the presence of the olefin.

## Description

### Field of the Invention

The present invention relates to a method for producing an internal olefin and to a method for regenerating the activity of a catalyst.

### Background of the Invention

Internal olefins are widely used as base oils for petroleum drilling oils, and raw materials for chemical products.

The internal olefin is produced by double bond isomerization or metathesis reaction using a 1-olefin as a raw material.

For example, JP 2008-517062 A (PTL 1) describes a method for isomerizing a 1-alkene to an internal alkene, including: a) combining at least one 1-alkene and a catalyst in a liquid phase at a temperature of about 50°C to about 200°C, in which the catalyst is formed by contacting (i) at least one Group 8 transition metal salt and (ii) at least one alkylaluminum compound to form a first mixture; and b) combining the first mixture of step a) with at least one acid washed clay at a temperature of about 100°C to about 300°C to form a final mixture.

In addition, JP 2016-539128 A (PTL 2) describes a method for isomerizing an olefin, the method including a step of bringing at least one olefin-containing raw material into contact with a catalyst exhibiting double-bond isomerizing activity on the olefin in a reaction zone to obtain an olefin isomerized product, in which the catalyst includes a γ-alumina-titania catalyst having a surface area exceeding 200 m²/g.

### Summary of the Invention

The present invention relates to a method for producing an internal olefin, including a step of subjecting a raw material olefin to an isomerization reaction in the presence of the following catalyst R:
Catalyst R: a catalyst obtained by holding a catalyst, which has been subjected to an isomerization reaction of an olefin at a reaction temperature (T₁), at a temperature higher than the reaction temperature (T₁) (hereinafter also referred to as a "catalyst holding temperature (T₂)") in the presence of the olefin.

### Brief Description of the Drawing

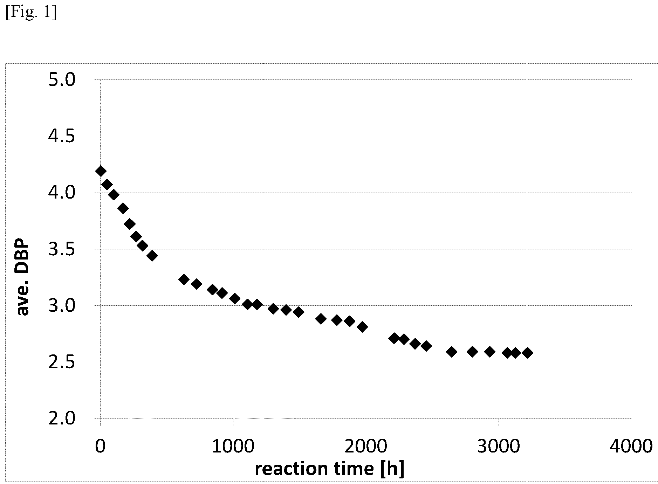

Fig. 1 is a diagram showing the change with time of the activity of a catalyst in the continuous reaction of step 1 of Example 1.

### Detailed Description of the Invention

In PTL 1, studies are made to obtain a final mixture containing an internal alkene and a low level of oligomer. In addition, in PTL 2, studies are made to improve resistance to an oxygen-containing compound as a poisoning material. However, in PTLs 1 and 2, there is room for further study on the problem that when a raw material olefin is industrially subjected to an isomerization reaction in the presence of a catalyst, the activity of the internal isomerization reaction of the catalyst decreases over time. Conventionally, a catalyst whose activity has been lowered through an isomerization reaction of a raw material olefin has been frequently discarded.

Hereinafter, the "activity of the internal isomerization reaction of the catalyst" is also simply referred to as the "activity of the catalyst".

Therefore, the present invention relates to a method for producing an internal olefin and a method for regenerating the activity of a catalyst, which are capable of regenerating the activity of a catalyst that has decreased due to an isomerization reaction of a raw material olefin.

The present inventors have found that the above problems can be solved by holding a catalyst, which has been subjected to an isomerization reaction of an olefin, under specific temperature conditions in the presence of the olefin. More specifically, the present inventors have found that it is possible to regenerate the activity of a catalyst by performing a treatment by temperature adjustment in the presence of a raw material olefin and/or in the presence of an internal olefin obtained by an isomerization reaction of the raw material olefin while there is a concern about catalyst deactivation due to multimerization or coking of the raw material olefin, or further catalyst deactivation due to a poisoning material in the raw material.

That is, the present invention relates to a method for producing an internal olefin, including a step of subjecting a raw material olefin to an isomerization reaction in the presence of the following catalyst R:
Catalyst R: a catalyst obtained by holding a catalyst, which has been subjected to an isomerization reaction of an olefin at a reaction temperature (T₁), at a temperature higher than the reaction temperature (T₁) (hereinafter also referred to as a "catalyst holding temperature (T₂)") in the presence of the olefin.

The present invention also relates to a method for regenerating the activity of a catalyst, including the following step:
a step of holding the catalyst, which has been subjected to an isomerization reaction of an olefin at a reaction temperature (T₁), at a catalyst holding temperature (T₂) higher than the reaction temperature (T₁) in the presence of the olefin.

According to the present invention, a method for producing an internal olefin and a method for regenerating the activity of a catalyst, which are capable of regenerating the activity of a catalyst that has decreased due to an isomerization reaction of a raw material olefin, are provided.

### [Method for Producing Internal Olefin]

The method for producing an internal olefin of the present invention is a method for producing an internal olefin, including a step of subjecting a raw material olefin to an isomerization reaction in the presence of the following catalyst R:
Catalyst R: a catalyst obtained by holding a catalyst, which has been subjected to an isomerization reaction of an olefin at a reaction temperature (T₁), at a temperature higher than the reaction temperature (T₁) (hereinafter also referred to as a "catalyst holding temperature (T₂)") in the presence of the olefin.

Here, "in the presence of an olefin" means in the presence of a raw material olefin and/or in the presence of an internal olefin obtained by an isomerization reaction of the raw material olefin.

The catalyst R may be a catalyst obtained by holding a catalyst, which has been subjected to an isomerization reaction of a raw material olefin at a reaction temperature (T₁), at a catalyst holding temperature (T₂) higher than the reaction temperature (T₁) in the presence of the raw material olefin.

Hereinafter, a step of obtaining a catalyst, which has been subjected to an isomerization reaction of an olefin at a reaction temperature (T₁), is referred to as Step 1, a step of obtaining a catalyst R by holding the catalyst obtained in Step 1 at a catalyst holding temperature (T₂) higher than the reaction temperature (T₁) in the presence of the olefin is referred to as Step 2, and a step of performing an isomerization reaction of a raw material olefin in the presence of the catalyst R is referred to as Step 3.

According to the production method of the present invention, there is an effect that the activity of the catalyst decreased by the isomerization reaction of the raw material olefin can be regenerated. The reason for this is not necessarily clear, but is considered as follows.

It is considered that with the progress of the isomerization reaction of the raw material olefin, a poisoning component is accumulated on the surface of the catalyst, and the activity of the catalyst decreases over time. However, it is considered that by holding the catalyst subjected to the isomerization reaction of the raw material olefin under a specific temperature condition, the poisoning component accumulated on the surface of the catalyst is removed for some reason, and the activity of the catalyst is regenerated.

It should be noted that the above-described mechanism relating to the effects of the present invention is a presumption and is not limited thereto.

Hereinafter, the raw material olefin and the catalyst used in the present invention will be described, and Step 1 (a step of obtaining a catalyst subjected to an isomerization reaction), Step 2 (a catalyst regeneration step), and Step 3 (an isomerization reaction step) will be described in detail.

### [Raw Material Olefin]

From the viewpoint of reactivity, the raw material olefin is preferably an olefin having 8 or more and 36 or less carbon atoms. The number of carbon atoms of the raw material olefin is preferably 8 or more, more preferably 10 or more, and further preferably 12 or more, and is also preferably 24 or less, more preferably 22 or less, and further preferably 18 or less from the viewpoint of the usefulness of the obtained internal olefin.

Examples of the raw material olefin include octene, nonene, decene, undecene, dodecene, tridecene, tetradecene, pentadecene, hexadecene, heptadecene, octadecene, nonadecene, eicosene, heneicocene, docosene, tricocene, and tetracocene, and from the viewpoint of availability, at least one selected from hexadecene and octadecene is preferable.

The double bond position of the raw material olefin is not particularly limited, and is preferably 1 or more and 4 or less, more preferably 1 or more and 3 or less, and further preferably 1 or 2, from the viewpoint of availability. That is, the raw material olefin is preferably a 1-olefin, a 2-olefin, a 3-olefin, or a 4-olefin, more preferably a 1-olefin, a 2-olefin, or a 3-olefin, and further preferably a 1-olefin or a 2-olefin. The raw material olefin may be a mixture of two or more kinds of olefins having different double bond positions.

The average double bond position of the raw material olefin is preferably 3.0 or less, more preferably 2.0 or less, and further preferably 1.8 or less, and is also preferably 1.0 or more, from the viewpoint of availability.

From the viewpoint of availability, it is preferable that the raw material olefin contains at least 1-olefin, and the 1-olefin is preferably a linear 1-olefin.

The raw material olefin may be obtained by any method, and a commercially available olefin having 8 or more and 36 or less carbon atoms, preferably 1-olefin having 8 or more and 36 or less carbon atoms may be used as the raw material olefin, or the raw material olefin may be produced by a dehydration reaction using an aliphatic primary alcohol having 8 or more and 36 or less carbon atoms (linear aliphatic primary alcohol having 8 or more and 36 or less carbon atoms) as a raw material (raw material alcohol). The reaction is represented by the following reaction formulas.

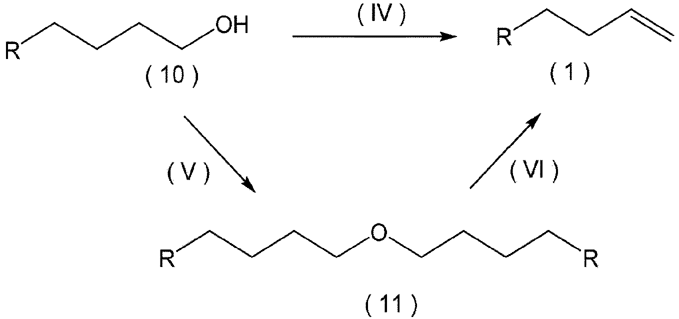

Here, R represents an alkyl group having 4 or more carbon atoms, and from the viewpoint of usefulness, R is preferably a linear alkyl group having 4 or more carbon atoms.

An aliphatic primary alcohol (raw material alcohol) (10) produces a 1-olefin (1) by intramolecular dehydration represented by (IV) and also produces a 1-olefin (1) via a reaction intermediate dialkyl ether (11) by intermolecular dehydration as shown in (V) and (VI). A part of the produced 1-olefin (1) becomes an internal olefin having a double bond at a position close to the terminal, such as 2-olefin, by an isomerization reaction.

The raw material alcohol may be any of an alcohol derived from petroleum and an alcohol derived from a natural raw material.

Examples of the naturally derived aliphatic primary alcohol include those derived from coconut oil, palm oil, palm kernel oil, soybean oil, rapeseed oil, beef tallow, lard, tall oil, and fish oil as raw materials.

Examples of the aliphatic primary alcohol include n-butanol, n-pentanol, n-hexanol, n-heptanol, n-octanol, n-nonanol, n-decanol, n-undecanol, n-dodecanol, n-tridecanol, n-tetradecanol, n-pentadecanol, n-hexadecanol, n-heptadecanol, n-octadecanol, n-nonadecanol, n-eicosanol, n-heneicosanol, n-docosanol, n-tricosanol, and n-tetracosanol, and among these, n-hexadecanol and n-octadecanol are preferable.

The method for producing the raw material olefin from the raw material alcohol is not particularly limited, and the raw material olefin may be produced by a known method, and is preferably produced by a liquid phase dehydration reaction in the presence of a solid catalyst, preferably in the presence of a solid acid catalyst, and the solid catalyst is preferably a metal oxide containing aluminum.

In addition, the reaction temperature of the liquid phase dehydration reaction is preferably 255°C or higher and 300°C or lower. In addition, the reaction pressure is preferably normal pressure.

### [Catalyst]

In the present invention, a catalyst which has been subjected to an olefin isomerization reaction step (Step 1) at a reaction temperature (T₁) is subjected to a catalyst regeneration step (Step 2) in which the catalyst is held at a temperature (catalyst holding temperature (T₂)) higher than the reaction temperature (T₁) in the presence of an olefin. Further, the catalyst (catalyst R) obtained by being subjected to the catalyst regeneration step (Step 2) is subjected to an isomerization reaction step (Step 3) of the raw material olefin.

From the viewpoint of reactivity, the catalyst used in Step 1 preferably contains an element of the third period to the fifth period, more preferably contains at least one selected from aluminum (Al), silicon (Si), titanium (Ti), iron (Fe), zinc (Zn), yttrium (Y), zirconium (Zr), and tin (Sn), and further preferably contains aluminum (Al).

From the viewpoint of reactivity and from the viewpoint of ease of operation, the catalyst is preferably a solid catalyst, more preferably a solid acid catalyst, and further preferably a solid Lewis acid catalyst.

Specifically, the catalyst is preferably at least one selected from an aluminum oxide catalyst (hereinafter, also simply referred to as "aluminum oxide"), an aluminum phosphate catalyst, and a zeolite (aluminosilicate) catalyst, and more preferably an aluminum oxide catalyst.

Examples of the crystalline form of the aluminum oxide (Al₂O₃) include γ, δ, and θ, and from the viewpoint of reactivity, the aluminum oxide is preferably γ-alumina.

From the viewpoint of reactivity, the purity of the aluminum oxide in the aluminum oxide catalyst is preferably 80% by mass or more, more preferably 90% by mass or more, further preferably 95% by mass or more, and still further preferably 98% by mass or more, and the upper limit is not particularly limited and is 100% by mass.

The reaction system in the isomerization reaction of the olefin is not particularly limited, may be a heterogeneous system or a homogeneous system, and is preferably a heterogeneous system from the viewpoint of efficiently subjecting the catalyst obtained in Step 1 to Step 2 and from the viewpoint of efficiently recovering the internal olefin produced by Step 1. Here, the homogeneous system means a system in which the catalyst and the olefin are compatible with each other, and the heterogeneous system means a system in which the catalyst and the olefin are incompatible with each other.

When the reaction system in the isomerization reaction of an olefin in Step 1 is a heterogeneous system, the catalyst in the present invention is preferably a solid catalyst.

As the solid catalyst, a powdery solid catalyst may be used as it is, or may be used as a granulated product or a molded product. Examples of the shape of the solid catalyst include a powder shape, a granular shape, a bead shape, a noodle shape, and a pellet shape. The shape of the solid catalyst is preferably a molded product such as a granular shape, a bead shape, a noodle shape, or a pellet shape, more preferably a bead shape or a noodle shape, and further preferably a bead shape, from the viewpoint of solid-liquid separability in a suspended bed batch reaction and from the viewpoint of a decrease in pressure loss in a fixed bed continuous reaction.

When the shape of the solid catalyst is a powder shape, the average particle diameter of the solid catalyst is preferably 1 µm or more, more preferably 3 µm or more, and further preferably 5 µm or more, and is also preferably 300 µm or less, more preferably 200 µm or less, further preferably 100 µm or less, and still further preferably 30 µm or less, from the viewpoint of the reactivity.

The average particle diameter of the powdery solid catalyst can be determined by a method such as a laser diffraction/scattering method.

When the shape of the solid catalyst is a granular shape, the average particle diameter of the solid catalyst is preferably 0.2 mm or more, more preferably 0.4 mm or more, and further preferably 0.6 mm or more, and is also preferably 2.0 mm or less, more preferably 1.3 mm or less, and further preferably 0.8 mm or less, from the viewpoint of the reactivity.

The average particle diameter of the granular solid catalyst can be determined by the following method.

That is, vibration was performed for 5 minutes using sieves of 2000 µm, 1400 µm, 1000 µm, 710 µm, 500 µm, 355 µm, 250 µm, 180 µm, 125 µm, 90 µm, 63 µm, and 45 µm specified in JIS Z 8801-1:2006 (established on May 20, 2000 and last amended on November 20, 2006), then 50% average diameter was calculated for a mass distribution under the sieves by the sieving method, and the 50% average diameter was used as the average particle diameter. To be specific, sieves of 2000 µm, 1400 µm, 1000 µm, 710 µm, 500 µm, 355 µm, 250 µm, 180 µm, 125 µm, 90 µm, 63 µm, and 45 µm specified in JIS Z 8801-1:2006 (established on May 20, 2000 and last amended on November 20, 2006) are stacked on a receiving tray in order from a sieve having a small mesh opening, 100 g of granules are added from the top of the uppermost sieve of 2000 µm, the sieve is covered with a lid, the sieve set is attached to a Ro-Tap sieve shaking machine (manufactured by HEIKO Seisakusho Co., Ltd., tapping: 156 times/min, rolling: 290 times/min), vibration is performed for 5 minutes, the mass of the granules remaining on each sieve and the receiving tray is measured, and the mass ratio (%) of the granules on each sieve is calculated. The mass ratio of the granules on the sieve with the smaller mesh opening is integrated in order from the receiving tray, and the particle diameter at which the total is 50% is taken as the average particle diameter.

When the shape of the solid catalyst is a bead shape, the diameter of the solid catalyst is preferably 1 mm or more, more preferably 1.5 mm or more, and further preferably 2 mm or more, and is also preferably 5 mm or less, more preferably 4 mm or less, and further preferably 3 mm or less, from the viewpoint of reactivity and from the viewpoint of easy recovery of the catalyst. The diameter of the solid catalyst may be an average value of any three measurements.

The diameter of the bead-shaped solid catalyst can be measured with a caliper.

When the shape of the solid catalyst is a noodle shape, the diameter of the solid catalyst is preferably 0.3 mm or more, more preferably 0.5 mm or more, and further preferably 0.7 mm or more, and is also preferably 2.5 mm or less, more preferably 2.3 mm or less, and further preferably 2.0 mm or less, from the viewpoint of reactivity and from the viewpoint of catalytic strength. Here, the diameter of the solid catalyst may be an average value measured at positions of 1/4, 1/2, and 3/4 of the length from one end in the longitudinal direction.

In addition, when the shape of the solid catalyst is a noodle shape, the length of the solid catalyst is preferably 2 mm or more, more preferably 2.5 mm or more, and further preferably 3 mm or more, and is also preferably 8 mm or less, more preferably 6 mm or less, and further preferably 5.5 mm or less, from the viewpoint of uniformly filling the catalyst in the reaction system and from the viewpoint of catalytic strength.

The diameter and length of the noodle-shaped solid catalyst can be measured with a caliper.

When the shape of the solid catalyst is a pellet shape, the diameter and length of the solid catalyst is preferably 1.5 mm or more, more preferably 2.0 mm or more, and further preferably 2.5 mm or more, and is also preferably 5.0 mm or less, more preferably 4.0 mm or less, and further preferably 3.0 mm or less, from the viewpoint of reactivity and from the viewpoint of catalytic strength.

The diameter and length of the pellet-shaped solid catalyst can be measured with a caliper.

The specific surface area of the solid catalyst is preferably 80 m²/g or more, more preferably 100 m²/g or more, and further preferably 120 m²/g or more, and is also preferably 300 m²/g or less, more preferably 280 m²/g or less, and further preferably 250 m²/g or less, from the viewpoint of reactivity and from the viewpoint of reaction selectivity.

The specific surface area of the solid catalyst can be generally measured by a method for determining a specific surface area, such as a BET method, and can be measured, for example, by a nitrogen adsorption method using a specific surface area measuring apparatus.

The average pore diameter of the solid catalyst is preferably 4 nm or more, more preferably 5 nm or more, and further preferably 6 nm or more, and is also preferably 20 nm or less, more preferably 17 nm or less, and further preferably 15 nm or less, from the viewpoint of reactivity and from the viewpoint of reaction selectivity.

For the average pore diameter of the solid catalyst, for example, a sample is subjected to heating pretreatment at 250°C for 5 hours, and then the pore diameter (diameter of pores) at the peak top of the pore diameter distribution calculated by the BJH method (Barrett-Joyner-Halenda method) by using a specific surface area/pore distribution measurement apparatus, can be taken as the average pore diameter. Here, the BJH method is a method in which calculation is performed using a cylindrical pore which is not connected to other pores as a model, and a pore distribution is obtained from capillary condensation and multi-molecular layer adsorption of nitrogen gas. Details thereof are described in "Shimadzu Review" (Vol. 48, No. 1, pp. 35-44, published in 1991).

The pore volume of the solid catalyst is preferably 0.1 mL/g or more, more preferably 0.15 mL/g or more, and further preferably 0.2 mL/g or more, and is also preferably 0.8 mL/g or less, more preferably 0.75 mL/g or less, and further preferably 0.7 mL/g or less, from the viewpoint of reactivity and from the viewpoint of reaction selectivity.

The pore volume of the solid catalyst can be measured by a general method for determining a pore volume, such as a mercury press-in method, and can be measured by, for example, a mercury press-in method using a mercury porosimeter.

The acid amount of the solid catalyst is preferably 0.1 mmol/g or more, more preferably 0.15 mmol/g or more, and further preferably 0.2 mmol/g or more, and is also preferably 1.5 mmol/g or less, more preferably 1.0 mmol/g or less, further preferably 0.9 mmol/g or less, still further preferably 0.85 mmol/g or less, and yet still further preferably 0.8 mmol/g or less, from the viewpoint of reactivity and from the viewpoint of reaction selectivity.

The acid content of the solid catalyst can be measured by a general method for determining the acid content, such as ammonia temperature-programmed desorption (NH₃-TPD).

The crushing strength of the solid catalyst is preferably 1 daN or more, more preferably 1.5 daN or more, and further preferably 2 daN or more, and is also preferably 11 daN or less, more preferably 10 daN or less, and further preferably 9 daN or less, from the viewpoint of catalytic strength.

The crushing strength of the solid catalyst can be measured, for example, by applying a compressive force to a granulated or molded catalyst using a fracture tester. The crushing pressure in the description herein is indicated by the magnitude of a force at the time when a crack starts to occur in the catalyst when a compressive force is applied to the granulated or molded catalyst.

The amount of the catalyst used is preferably adjusted as appropriate depending on the reaction system.

In a case where the reaction type is a batch type, the amount of the catalyst to be used is preferably 0.5 parts by mass or more, more preferably 1 part by mass or more, and further preferably 2 parts by mass or more, and is also preferably 50 parts by mass or less, more preferably 40 parts by mass or less, and further preferably 35 parts by mass or less, with respect to 100 parts by mass of the raw material olefin, from the viewpoint of the reactivity.

In a case where the reaction type is a continuous type, the amount of the catalyst to be used can be appropriately adjusted depending on the amount of the raw material olefin to be supplied (liquid feeding amount).

### [Step 1: Step of Obtaining Catalyst Subjected to Isomerization Reaction]

Step 1 is a step of obtaining a catalyst subjected to an isomerization reaction of olefin at reaction temperature (T₁). The olefin may be the raw material olefin described above, and the isomerization reaction in Step 1 may be an internal isomerization reaction, or may include an isomerization reaction in which a 2-olefin becomes a 1-olefin.

### (Reaction Type)

The reaction system in Step 1 is not particularly limited, and may be a heterogeneous system or a homogeneous system, and from the viewpoint of efficiently subjecting the catalyst subjected to Step 1 to Step 2 and from the viewpoint of ease of operation, a heterogeneous system is preferred.

The reaction type of the isomerization reaction in Step 1 is not particularly limited, a batch type or a continuous type can be appropriately selected, and from the viewpoint of ease of operation, a continuous type is preferably selected.

In addition, in a case where the reaction type is a continuous type, any of a fixed bed method and a suspension bed method may be adopted. Among these, a fixed bed method is preferable from the viewpoint of ease of operation. That is, the reaction type of the isomerization reaction in Step 1 is preferably a continuous fixed bed method.

### (Reaction Temperature T₁)

When the isomerization reaction temperature in Step 1 is represented by T₁ (°C), the reaction temperature T₁ in Step 1 is preferably 120°C or higher, more preferably 140°C or higher, and further preferably 160°C or higher, and is also preferably 340°C or lower, more preferably 320°C or lower, and further preferably 300°C or lower, from the viewpoint of reactivity and from the viewpoint of reaction selectivity.

### (Reaction Pressure)

The pressure in the reactor during the reaction is not particularly limited, and is preferably 1 MPa or less, more preferably 0.5 MPa or less, further preferably 0.2 MPa or less, and still further preferably 0.1 MPa, i.e., atmospheric pressure, in terms of absolute pressure, from the viewpoint of safety and ease of operation.

### (Reaction Atmosphere)

During the reaction, an inert gas can be introduced into the reactor.

Examples of the inert gas include nitrogen, argon, and helium, and nitrogen is preferable from the viewpoint of availability. The inert gas is preferably circulated as a carrier.

The flow rate of the inert gas can be appropriately adjusted depending on the scale of the reactor and the reaction type, and is preferably 0.5 NL/hr or more, more preferably 1 NL/hr or more, and further preferably 3 NL/hr or more, and is also preferably 20 NL/hr or less, more preferably 10 NL/hr or less, and further preferably 5 NL/hr or less.

When the reaction type is a batch type, the flow of the inert gas can be performed by a method of flowing the inert gas above the reaction solution, or a method of bubbling the inert gas in the reaction solution.

### (Reaction Time)

The reaction time in the isomerization reaction is appropriately determined depending on the type and supply amount of the (raw material) olefin and the reaction temperature.

In a case where the reaction type is a batch type, from the viewpoint of reactivity and reaction selectivity, the reaction time is preferably 0.25 hr or more, more preferably 0.5 hr or more, and further preferably 1.0 hr or more, and is also preferably 20 hr or less, more preferably 16 hr or less, further preferably 12 hr or less, still further preferably 8 hr or less, and yet still further preferably 5 hr or less.

### (Rotation Speed of Stirring)

When the reaction type is a batch type, the rotation speed of stirring can be appropriately set depending on the scale of the reactor, and is preferably 20 rpm or more, more preferably 100 rpm or more, and further preferably 300 rpm or more, and is also preferably 800 rpm or less, more preferably 700 rpm or less, and further preferably 600 rpm or less.

### (LHSV (Liquid Hourly Space Velocity))

When the reaction type is a continuous type, the LHSV (liquid hourly space velocity) representing the supply amount of the (raw material) olefin relative to the amount of the catalyst to be used is preferably 0.05/hr or more, more preferably 0.1/hr or more, and further preferably 0.15/hr or more, and is also preferably 20/hr or less, more preferably 15/hr or less, and further preferably 10/hr or less, from the viewpoint of the reactivity and from the viewpoint of the reaction selectivity.

### (Catalyst Obtained in Step 1)

When the reaction type is a batch type, the catalyst obtained in Step 1 is appropriately separated from the reaction system and subjected to the catalyst regeneration step of Step 2. On the other hand, when the reaction type is a continuous type, the catalyst obtained in Step 1 can be subjected to the catalyst regeneration step of Step 2 without requiring an operation of separating the catalyst from the reaction system.

### [Step 2: Catalyst Regeneration Step]

Step 2 is a step of holding the catalyst obtained in Step 1 described above at a catalyst-holding temperature (T₂) higher than the reaction temperature (T₁) to obtain catalyst R.

From the viewpoint of catalyst regeneration efficiency, Step 2 is performed in the presence of an olefin, and is preferably performed under a flow of the olefin. The olefin used in Step 2 may be the above-described raw material olefin, and may not be the same as the olefin used in Steps 1 and 3, but is preferably an olefin of the same type or having the same composition as the raw material olefin used in Step 3, from the viewpoint of producing an internal olefin with less impurities. In addition, when the catalyst regeneration step is performed in the presence of the raw material olefin used in Step 3, a special washing operation of the catalyst and the equipment after the catalyst regeneration step is not required, which is preferable.

### (Catalyst Holding Type)

The catalyst holding system in Step 2 is not particularly limited, may be a heterogeneous system or a homogeneous system as in Step 1, and is preferably a heterogeneous system from the viewpoint of efficiently regenerating the activity of the catalyst decreased by the isomerization reaction of the (raw material) olefin in Step 1.

The catalyst holding type in Step 2 is not particularly limited, and as in Step 1, a batch type or a continuous type can be appropriately selected. From the viewpoint of efficiently regenerating the activity of the catalyst decreased by the isomerization reaction of the (raw material) olefin in Step 1, the continuous type is preferably selected.

In addition, in a case where the catalyst holding type is a continuous type, any of a fixed bed method and a suspension bed method may be adopted. Among these, a fixed bed method is preferable from the viewpoint of ease of operation. That is, the catalyst holding type in Step 2 is preferably a continuous fixed bed method.

### (Catalyst Holding Temperature T₂)

In Step 2, the catalyst obtained in Step 1 is held at a temperature higher than the reaction temperature T₁ of Step 1 from the viewpoint of efficiently regenerating the activity of the catalyst decreased by the isomerization reaction of the olefin in Step 1.

When the catalyst holding temperature in Step 2 at which the catalyst obtained in Step 1 is held at a temperature higher than the reaction temperature T₁ in Step 1 is represented by T₂ (°C), the catalyst holding temperature T₂ in Step 2 is preferably a temperature higher by 5°C or more, more preferably a temperature higher by more than 30°C, further preferably a temperature higher by 35°C or more, still further preferably a temperature higher by 40°C or more, and yet still further preferably a temperature higher by 45°C or more than the reaction temperature T₁ in Step 1, from the viewpoint of efficiently regenerating the activity of the catalyst decreased by the isomerization reaction of the olefin in Step 1.

That is, the difference ΔT(T₂ - T₁) between the catalyst holding temperature (T₂) in Step 2 and the reaction temperature (T₁) in Step 1 is preferably 5°C or higher, more preferably higher than 30°C, further preferably 35°C or higher, still further preferably 40°C or higher, and yet still further preferably 45°C or higher, and is also preferably 150°C or lower, more preferably 130°C or lower, further preferably 100°C or lower, and still further preferably 80°C or lower, from the viewpoint of efficiently regenerating the activity of the catalyst decreased by the isomerization reaction of the (raw material) olefin in Step 1.

The catalyst-holding temperature (T₂) in Step 2 may be appropriately selected in relation to the reaction temperature (T₁) in Step 1 so that ΔT(T₂ - T₁) falls within the above range, and is preferably 180°C or higher, more preferably 200°C or higher, and further preferably 210°C or higher, and is also preferably 350°C or lower, more preferably 330°C or lower, and further preferably 320°C or lower, from the viewpoint of efficiently regenerating the activity of the catalyst decreased by the isomerization reaction of the (raw material) olefin in Step 1.

When the reaction temperature (T₁) varies, the reaction temperature (T₁) may be an average value. Further, when the catalyst holding temperature (T₂) varies, the catalyst holding temperature (T₂) may be an average value.

Examples of the method for measuring the reaction temperature (T₁) include a contact-type measurement method using a thermocouple. In addition, examples of the method for measuring the catalyst holding temperature (T₂) include a contact-type measurement method using a thermocouple.

### (Catalyst Holding Pressure)

The pressure in the reactor used in the catalyst holding step is not particularly limited, and is preferably 1 MPa or less, more preferably 0.5 MPa or less, further preferably 0.2 MPa or less, and still further preferably 0.1 MPa, i.e., atmospheric pressure, in terms of absolute pressure, from the viewpoint of safety and ease of operation.

### (Catalyst Holding Atmosphere)

In the catalyst holding, an inert gas can be introduced into the reactor.

Examples of the inert gas include nitrogen, argon, and helium, and nitrogen is preferable from the viewpoint of availability. The inert gas is preferably circulated as a carrier.

The flow rate of the inert gas can be appropriately adjusted depending on the scale of the reactor and the reaction type, and is preferably 0.01 NL/hr or more, more preferably 1 NL/hr or more, and further preferably 3 NL/hr or more, and is also preferably 20 NL/hr or less, more preferably 10 NL/hr or less, and further preferably 5 NL/hr or less.

When the reaction type is a batch type, the flow of the inert gas can be performed by a method of flowing the inert gas above the reaction solution, or a method of bubbling the inert gas in the reaction solution.

### (Catalyst Holding Time)

The catalyst holding time in the catalyst regeneration step is appropriately determined according to the catalyst holding temperature (T₂) or the like.

When the catalyst holding type is a batch type, the catalyst holding time is preferably 0.25 hr or more, more preferably 0.5 hr or more, and further preferably 1.0 hr or more, and is also preferably 20 hr or less, more preferably 16 hr or less, further preferably 12 hr or less, and still further preferably 10 hr or less, from the viewpoint of efficiently regenerating the activity of the catalyst decreased by the isomerization reaction of the (raw material) olefin in Step 1.

### (Rotation Speed of Stirring)

When the catalyst holding type is a batch type, the rotation speed of stirring can be appropriately set depending on the scale of the reactor, and is preferably 20 rpm or more, more preferably 100 rpm or more, and further preferably 300 rpm or more, and is also preferably 800 rpm or less, more preferably 700 rpm or less, and further preferably 600 rpm or less.

### (LHSV (Liquid Hourly Space Velocity))

When the catalyst holding type is a continuous type, the LHSV (liquid hourly space velocity) representing the supply amount of the (raw material) olefin relative to the amount of the catalyst to be used is preferably 0.05/hr or more, more preferably 0.1/hr or more, and further preferably 0.15/hr or more, and is also preferably 20/hr or less, more preferably 15/hr or less, and further preferably 10/hr or less, from the viewpoint of efficiently regenerating the activity of the catalyst decreased by the isomerization reaction of the (raw material) olefin in Step 1.

### (Catalyst Obtained in Step 2)

When the catalyst holding type is a batch type, the catalyst obtained in Step 2 is appropriately separated from the catalyst holding system and subjected to the isomerization reaction step of Step 3. On the other hand, when the catalyst holding type is a continuous type, the catalyst obtained in Step 2 can be subjected to the isomerization reaction step of Step 3 without requiring an operation of separating the catalyst from the catalyst holding system.

### (Step 3: Isomerization Reaction Step)

Step 3 is a step of subjecting a raw material olefin to an isomerization reaction in the presence of the catalyst (catalyst R) obtained in Step 2. The isomerization reaction in Step 3 may be an internal isomerization reaction as a main reaction, or may include an isomerization reaction in which a 2-olefin becomes a 1-olefin.

### (Reaction Type)

The reaction system in Step 3 is not particularly limited, may be a heterogeneous system or a homogeneous system as in Steps 1 and 2, and is preferably a heterogeneous system from the viewpoint of ease of operation.

The reaction type of the isomerization reaction in Step 3 is not particularly limited, and a batch type or a continuous type can be appropriately selected as in Steps 1 and 2, but from the viewpoint of ease of operation, a continuous type is preferably selected.

In addition, in a case where the reaction type is a continuous type, any of a fixed bed method and a suspension bed method may be adopted. Among these, a fixed bed method is preferable from the viewpoint of ease of operation. That is, the reaction type of the isomerization reaction in Step 3 is preferably a continuous fixed bed method.

### (Reaction Temperature T₃)

When the isomerization reaction temperature in Step 3 is represented by T₃ (°C), the isomerization reaction temperature T₃ in Step 3 is preferably lower than the catalyst holding temperature T₂ in Step 2 from the viewpoint of reactivity and from the viewpoint of reaction selectivity. Further, from the viewpoint of reactivity and from the viewpoint of reaction selectivity, the reaction temperature T₃ in Step 3 is preferably a temperature lower by 5°C or more, more preferably a temperature lower by 10°C or more, and further preferably a temperature lower by 15°C or more, than the catalyst holding temperature T₂ in Step 2.

That is, the difference ΔT(T₂ - T₃) between the catalyst holding temperature (T₂) in Step 2 and the reaction temperature (T₃) in Step 3 is preferably 5°C or higher, more preferably 10°C or higher, and further preferably 15°C or higher, and is also preferably 150°C or lower, more preferably 130°C or lower, and further preferably 120°C or lower, from the viewpoint of reactivity and from the viewpoint of reaction selectivity.

The reaction temperature (T₃) in Step 3 may be appropriately selected in relation to the catalyst holding temperature (T₂) in Step 2 so that ΔT(T₂ - T₃) falls within the above range, and is preferably 120°C or higher, more preferably 140°C or higher, and further preferably 160°C or higher, and is also preferably 340°C or lower, more preferably 320°C or lower, and further preferably 300°C or lower, from the viewpoint of reactivity and from the viewpoint of reaction selectivity.

When the reaction temperature (T₃) in Step 3 varies, the reaction temperature (T₃) may be an average value.

The method for measuring the reaction temperature (T₃) is the same as that for the reaction temperature (T₁).

### (Reaction Pressure)

The pressure in the reactor during the reaction is not particularly limited, and is preferably 1 MPa or less, more preferably 0.5 MPa or less, further preferably 0.2 MPa or less, and still further preferably 0.1 MPa, i.e., atmospheric pressure, in terms of absolute pressure, from the viewpoint of safety and ease of operation.

### (Reaction Atmosphere)

During the reaction, an inert gas can be introduced into the reactor.

Examples of the inert gas include nitrogen, argon, and helium, and nitrogen is preferable from the viewpoint of availability. The inert gas is preferably circulated as a carrier.

The flow rate of the inert gas can be appropriately adjusted depending on the scale of the reactor and the reaction type, and is preferably 0.01 NL/hr or more, more preferably 1 NL/hr or more, and further preferably 3 NL/hr or more, and is also preferably 20 NL/hr or less, more preferably 10 NL/hr or less, and further preferably 5 NL/hr or less.

When the reaction type is a batch type, the flow of the inert gas can be performed by a method of flowing the inert gas above the reaction solution, or a method of bubbling the inert gas in the reaction solution.

### (Reaction Time)

The reaction time in the isomerization reaction is appropriately determined depending on the type and supply amount of the raw material olefin and the reaction temperature.

In a case where the reaction type is a batch type, from the viewpoint of reactivity and from the viewpoint of reaction selectivity, the reaction time is preferably 0.25 hr or more, more preferably 0.5 hr or more, and further preferably 1.0 hr or more, and is also preferably 20 hr or less, more preferably 16 hr or less, further preferably 12 hr or less, still further preferably 10 hr or less, and yet still further preferably 5 hr or less.

### (Rotation Speed of Stirring)

When the reaction type is a batch type, the rotation speed of stirring can be appropriately set depending on the scale of the reactor, and is preferably 20 rpm or more, more preferably 100 rpm or more, and further preferably 300 rpm or more, and is also preferably 800 rpm or less, more preferably 700 rpm or less, and further preferably 600 rpm or less.

### (LHSV (Liquid Hourly Space Velocity))

When the reaction type is a continuous type, the LHSV (liquid hourly space velocity) representing the supply amount of the raw material olefin relative to the amount of the catalyst to be used is preferably 0.05/hr or more, more preferably 0.1/hr or more, and further preferably 0.15/hr or more, and is also preferably 20/hr or less, more preferably 15/hr or less, and further preferably 10/hr or less, from the viewpoint of the reactivity and from the viewpoint of the reaction selectivity.

### (Separation of Internal Olefin)

When the reaction type is a batch type, the internal olefin produced in Step 3 is obtained by appropriately separating the internal olefin from the catalyst that has passed through Step 3. On the other hand, when the reaction type is a continuous type, the internal olefin produced in Step 3 can be obtained without requiring an operation of separating the internal olefin from the catalyst that has passed through Step 3.

### [Use of Internal Olefin]

The internal olefin obtained by the method for producing an internal olefin of the present invention is useful as a raw material or an intermediate raw material for a surfactant, an organic solvent, a softening agent, or a sizing agent, and is particularly useful as a raw material for a surfactant.

In addition to the above-described embodiments, the present invention discloses the following method for producing an internal olefin.
<1> A method for producing an internal olefin, including a step of subjecting a raw material olefin to an isomerization reaction in the presence of the following catalyst R:
   Catalyst R: a catalyst obtained by holding a catalyst, which has been subjected to an isomerization reaction of an olefin at a reaction temperature (T₁), at a temperature higher than the reaction temperature (T₁) (hereinafter also referred to as a "catalyst holding temperature (T₂)") in the presence of the olefin.
   Hereinafter, a step of obtaining a catalyst, which has been subjected to an isomerization reaction of an olefin at a reaction temperature (T₁), is referred to as Step 1, a step of obtaining a catalyst R by holding the catalyst obtained in Step 1 at a catalyst holding temperature (T₂) higher than the reaction temperature (T₁) is referred to as Step 2, and a step of performing an isomerization reaction of a raw material olefin in the presence of the catalyst R is referred to as Step 3.
<2> The method for producing an internal olefin according to <1>, in which the catalyst R is a catalyst obtained by holding a catalyst, which has been subjected to an isomerization reaction of an olefin at a reaction temperature (T₁), at a temperature higher than the reaction temperature (T₁) (hereinafter also referred to as a "catalyst holding temperature (T₂)") in the presence of a raw material olefin.
<3> The method for producing an internal olefin according to <1> or <2>, in which Step 2 is performed under a flow of the olefin.
<4> The method for producing an internal olefin according to any one of <1> to <3>, in which a difference ΔT(T₂ - T₁) between the catalyst holding temperature (T₂) in Step 2 and the reaction temperature (T₁) in Step 1 is preferably 5°C or higher, more preferably higher than 30°C, further preferably 35°C or higher, still further preferably 40°C or higher, and yet still further preferably 45°C or higher, and is also preferably 150°C or lower, more preferably 130°C or lower, further preferably 100°C or lower, and still further preferably 80°C or lower.
<5> The method for producing an internal olefin according to any one of <1> to <4>, in which the difference ΔT(T₂ - T₁) between the catalyst holding temperature (T₂) in Step 2 and the reaction temperature (T₁) in Step 1 is higher than 30°C and 150°C or lower.
<6> The method for producing an internal olefin according to any one of <1> to <5>, in which the catalyst holding temperature (T₂) in Step 2 is preferably 180°C or higher, more preferably 200°C or higher, and further preferably 210°C or higher, and is also preferably 350°C or lower, more preferably 330°C or lower, and further preferably 320°C or lower.
<7> The method for producing an internal olefin according to any one of <1> to <6>, in which the pressure in the reactor used in the catalyst holding step of Step 2 is, in terms of absolute pressure, preferably 1 MPa or less, more preferably 0.5 MPa or less, further preferably 0.2 MPa or less, and still further preferably 0.1 MPa, i.e., atmospheric pressure.
<8> The method for producing an internal olefin according to any one of <1> to <7>, in which Step 2 is performed under an inert gas flow.
<9> The method for producing an internal olefin according to <8>, in which the flow rate of the inert gas is preferably 0.01 NL/hr or more, more preferably 1 NL/hr or more, and further preferably 3 NL/hr or more, and is also preferably 20 NL/hr or less, more preferably 10 NL/hr or less, and further preferably 5 NL/hr or less.
<10> The method for producing an internal olefin according to any one of <1> to <9>, in which the catalyst holding type of Step 2 is a batch type.
<11> The method for producing an internal olefin according to <10>, in which the catalyst holding time in Step 2 is preferably 0.25 hr or more, more preferably 0.5 hr or more, and further preferably 1.0 hr or more, and is also preferably 20 hr or less, more preferably 16 hr or less, further preferably 12 hr or less, and still further preferably 10 hr or less.
<12> The method for producing an internal olefin according to <10> or <11>, in which the rotation speed of the stirring in Step 2 is preferably 20 rpm or more, more preferably 100 rpm or more, and further preferably 300 rpm or more, and is also preferably 800 rpm or less, more preferably 700 rpm or less, and further preferably 600 rpm or less.
<13> The method for producing an internal olefin according to any one of <1> to <9>, in which the catalyst holding type of Step 2 is a continuous type.
<14> The method for producing an internal olefin according to <13>, in which a LHSV (liquid hourly space velocity) representing a supply amount of the raw material olefin with respect to the amount of the catalyst used in Step 2 is preferably 0.05/hr or more, more preferably 0.1/hr or more, and further preferably 0.15/hr or more, and is also preferably 20/hr or less, more preferably 15/hr or less, and further preferably 10/hr or less.
<15> The method for producing an internal olefin according to any one of <1> to <14>, in which the reaction temperature (T₃) in Step 3 is lower than the catalyst holding temperature (T₂) in Step 2.
<16> The method for producing an internal olefin according to <15>, in which a difference ΔT(T₂ - T₃) between the catalyst holding temperature (T₂) in Step 2 and the reaction temperature (T₃) in Step 3 is preferably 5°C or higher, more preferably 10°C or higher, and further preferably 15°C or higher, and is also preferably 150°C or lower, more preferably 130°C or lower, and further preferably 120°C or lower.
<17> The method for producing an internal olefin according to <15> or <16>, in which the difference ΔT(T₂ - T₃) between the catalyst holding temperature (T₂) in Step 2 and the reaction temperature (T₃) in Step 3 is 10°C or higher and 130°C or lower.
<18> The method for producing an internal olefin according to any one of <15> to <17>, in which the reaction temperature (T₃) in Step 3 is preferably 120°C or higher, more preferably 140°C or higher, and further preferably 160°C or higher, and is also preferably 340°C or lower, more preferably 320°C or lower, and further preferably 300°C or lower.
<19> The method for producing an internal olefin according to any one of <15> to <18>, in which the pressure in the reactor of Step 3 is, in terms of absolute pressure, preferably 1 MPa or less, more preferably 0.5 MPa or less, further preferably 0.2 MPa or less, and still further preferably 0.1 MPa, i.e., atmospheric pressure.
<20> The method for producing an internal olefin according to any one of <15> to <19>, in which Step 3 is performed under an inert gas flow.
<21> The method for producing an internal olefin according to <20>, in which the flow rate of the inert gas is preferably 0.01 NL/hr or more, more preferably 1 NL/hr or more, and further preferably 3 NL/hr or more, and is also preferably 20 NL/hr or less, more preferably 10 NL/hr or less, and further preferably 5 NL/hr or less.
<22> The method for producing an internal olefin according to any one of <15> to <21>, in which the reaction type of Step 3 is a batch type.
<23> The method for producing an internal olefin according to <22>, in which the reaction time in Step 3 is preferably 0.25 hr or more, more preferably 0.5 hr or more, and further preferably 1.0 hr or more, and is also preferably 20 hr or less, more preferably 16 hr or less, further preferably 12 hr or less, still further preferably 10 hr or less, and yet still further preferably 5 hr or less.
<24> The method for producing an internal olefin according to <22> or <23>, in which the rotation speed of the stirring in Step 3 is preferably 20 rpm or more, more preferably 100 rpm or more, and further preferably 300 rpm or more, and is also preferably 800 rpm or less, more preferably 700 rpm or less, and further preferably 600 rpm or less.
<25> The method for producing an internal olefin according to any one of <15> to <21>, in which the reaction type of Step 3 is a continuous type.
<26> The method for producing an internal olefin according to <25>, in which a LHSV (liquid hourly space velocity) representing a supply amount of the raw material olefin with respect to the amount of the catalyst used in Step 3 is preferably 0.05/hr or more, more preferably 0.1/hr or more, and further preferably 0.15/hr or more, and is also preferably 20/hr or less, more preferably 15/hr or less, and further preferably 10/hr or less.
<27> The method for producing an internal olefin according to any one of <1> to <26>, in which the reaction temperature (T₁) in Step 1 is preferably 120°C or higher, more preferably 140°C or higher, and further preferably 160°C or higher, and is also preferably 340°C or lower, more preferably 320°C or lower, and further preferably 300°C or lower.
<28> The method for producing an internal olefin according to any one of <1> to <27>, in which the reaction temperature (T₁) in Step 1 is 160°C or higher and 300°C or lower.
<29> The method for producing an internal olefin according to any one of <1> to <28>, in which the pressure in the reactor of Step 1 is, in terms of absolute pressure, preferably 1 MPa or less, more preferably 0.5 MPa or less, further preferably 0.2 MPa or less, and still further preferably 0.1 MPa, i.e., atmospheric pressure.
<30> The method for producing an internal olefin according to <1> to <29>, in which Step 1 is performed under an inert gas flow.
<31> The method for producing an internal olefin according to <30>, in which the flow rate of the inert gas in Step 1 is preferably 0.5 NL/hr or more, more preferably 1 NL/hr or more, and further preferably 3 NL/hr or more, and is also preferably 20 NL/hr or less, more preferably 10 NL/hr or less, and further preferably 5 NL/hr or less.
<32> The method for producing an internal olefin according to <1> to <31>, in which the reaction type of Step 1 is a batch type.
<33> The method for producing an internal olefin according to <32>, in which the reaction time in Step 1 is preferably 0.25 hr or more, more preferably 0.5 hr or more, and further preferably 1.0 hr or more, and is also preferably 20 hr or less, more preferably 16 hr or less, further preferably 12 hr or less, still further preferably 8 hr or less, and yet still further preferably 5 hr or less.
<34> The method for producing an internal olefin according to <32> or <33>, in which the rotation speed of the stirring in Step 1 is preferably 20 rpm or more, more preferably 100 rpm or more, and further preferably 300 rpm or more, and is also preferably 800 rpm or less, more preferably 700 rpm or less, and further preferably 600 rpm or less.
<35> The method for producing an internal olefin according to <1> to <31>, in which the reaction type of Step 1 is a continuous type.
<36> The method for producing an internal olefin according to <35>, in which a LHSV (liquid hourly space velocity) representing a supply amount of the raw material olefin with respect to the amount of the catalyst used in Step 1 is preferably 0.05/hr or more, more preferably 0.1/hr or more, and further preferably 0.15/hr or more, and is also preferably 20/hr or less, more preferably 15/hr or less, and further preferably 10/hr or less.
<37> The method for producing an internal olefin according to any one of <1> to <36>, in which the catalyst is a solid Lewis acid catalyst.
<38> The method for producing an internal olefin according to any one of <1> to <37>, in which the catalyst preferably contains an element of the third period to the fifth period, more preferably contains at least one selected from aluminum (Al), silicon (Si), titanium (Ti), iron (Fe), zinc (Zn), yttrium (Y), zirconium (Zr), and tin (Sn), and further preferably contains aluminum (Al).
<39> The method for producing an internal olefin according to any one of <1> to <38>, in which the catalyst is preferably at least one selected from an aluminum oxide catalyst (hereinafter, also simply referred to as "aluminum oxide"), an aluminum phosphate catalyst, and a zeolite (aluminosilicate) catalyst, and more preferably an aluminum oxide catalyst.
<40> The method for producing an internal olefin according to <39>, in which the aluminum oxide is preferably γ-alumina, and the purity of the aluminum oxide in the aluminum oxide catalyst is preferably 80% by mass or more, more preferably 90% by mass or more, further preferably 95% by mass or more, and still further preferably 98% by mass or more, and the upper limit is not particularly limited and is 100% by mass.
<41> The method for producing an internal olefin according to any one of <1> to <40>, in which the double bond position of the raw material olefin is preferably 1 or more and 4 or less, more preferably 1 or more and 3 or less, and further preferably 1 or 2.
<42> The method for producing an internal olefin according to any one of <1> to <41>, in which the raw material olefin preferably contains a 1-olefin, and the 1-olefin is preferably a linear 1-olefin.
<43> The method for producing an internal olefin according to any one of <1> to <42>, in which the raw material olefin has preferably 8 or more carbon atoms, more preferably 10 or more carbon atoms, and further preferably 12 or more carbon atoms, and also preferably 24 or less carbon atoms, more preferably 22 or less carbon atoms, and further preferably 18 or less carbon atoms.
<44> The method for producing an internal olefin according to any one of <1> to <43>, in which the raw material olefin has 12 or more and 24 or less carbon atoms.
<45> A method for regenerating the activity of a catalyst, including the following step:
   a step of holding the catalyst, which has been subjected to an isomerization reaction of an olefin at a reaction temperature (T₁), at a catalyst holding temperature (T₂) higher than the reaction temperature (T₁) in the presence of the olefin.
<46> The method for regenerating the activity of a catalyst according to <45>, in which the step is a step of holding a catalyst, which has been subjected to an isomerization reaction of a raw material olefin at a reaction temperature (T₁), at a catalyst holding temperature (T₂) higher than the reaction temperature (T₁) in the presence of a raw material olefin.
<47> The method for regenerating the activity of a catalyst according to <45> or <46>, in which the step is preferably performed under a flow of the olefin.
<48> The method for regenerating the activity of a catalyst according to any one of <45> to <47>, in which the step is Step 2 in the method for producing an internal olefin according to any one of <1> to <44>.

### Examples

Hereinafter, the present invention will be specifically described by Examples, but the present invention is not limited to these Examples in any way.

### Example 1

### [Step 1: Isomerization Reaction Step]

In a catalyst continuous evaluation apparatus, 400 mL of a γ-alumina catalyst (trade name "Neobead GB-13", manufactured by Mizusawa Industrial Chemicals, Ltd., γ-Al₂O₃, bead shape, diameter of 2.0 mm, specific surface area of 180 m²/g, average pore diameter of 11.1 nm, pore volume of 0.50 mL/g, acid amount of 0.28 mmol/g, crushing strength of 2.6 daN) was charged as a catalyst, 1-hexadecene (manufactured by FUJIFILM Wako Pure Chemical Corporation) was sent as a raw material olefin at 100 mL/h, and a continuous reaction was carried out under the conditions of a nitrogen pressure of 0.1 MPaG, a reaction temperature (T₁) of 230°C, and a reaction time of 3,216 h while flowing nitrogen at 3.9 NL/h. After completion of the reaction, the catalyst layer was cooled to room temperature and depressurized, and then the catalyst was taken out from the catalyst continuous evaluation apparatus and used in the next step.

### <Quantification of Product>

In the continuous reaction of Step 1, the reaction solution was sampled from the outlet of the apparatus every 12 hours, about one drop of the sampled reaction solution was put into a sample tube, 1 mL of dimethyl disulfide (manufactured by Tokyo Chemical Industry Co., Ltd.) and 30 mg of iodine (manufactured by FUJIFILM Wako Pure Chemical Corporation) were added thereto, the components were sufficiently mixed, and the mixture was allowed to stand at 65°C for 15 minutes. Next, 1 mL of a 30% by mass aqueous solution of sodium thiosulfate (manufactured by FUJIFILM Wako Pure Chemical Corporation), 1 mL of ethanol (manufactured by FUJIFILM Wako Pure Chemical Corporation), and 2 mL of hexane (manufactured by FUJIFILM Wako Pure Chemical Corporation) were added and mixed, and then the upper phase was collected, a column "Ultra ALLOY-1" (manufactured by Frontier Laboratories Ltd.: capillary column: 30.0 m × 250 µm) was attached to a gas chromatograph analyzer "Agilent 6890A" (manufactured by Agilent Technology Corporation), analysis was performed using a hydrogen flame ion detector (FID) under the conditions of an injection temperature of 300°C, a detector temperature of 350°C, and a He flow rate of 0.8 mL/min, and the product was quantified. The average double bond position (Ave.DBP) was calculated from the olefin content at each double bond position according to the following formula 1. The change with time was shown in Fig. 1. From the results shown in Fig. 1, it was confirmed that the activity of the catalyst decreased over time due to the isomerization reaction of the raw material olefin in Step 1. Ave.DBP = (1-olefin + 2 × 2-olefin + 3 × 3-olefin + 4 × 4-olefin + 5 × 5-olefin + 6 × 6-olefin + 7 × 7-olefin + 8 × 8-olefin)/100

### <Catalytic Activity Evaluation>

In a catalyst continuous reaction apparatus different from the apparatus for Step 1, 10 mL of the catalyst that has passed through Step 1 was charged, 1-hexadecene (manufactured by FUJIFILM Wako Pure Chemical Corporation) was sent thereto at 80 mL/h, and a continuous reaction was performed under the conditions of a reaction temperature of 260°C and a reaction time of 6 h while flowing nitrogen at 1.9 NL/h. In the reaction solution collected at the end of the reaction, the amount of the product was determined by the above-described method, and the average double bond position (Ave.DBP₁) was calculated.

### [Step 2: Catalyst Regeneration Step]

1-Hexadecene (manufactured by FUJIFILM Wako Pure Chemical Corporation) was sent to the catalyst continuous reaction apparatus after the evaluation of the catalytic activity at 80 mL/h, and a continuous reaction was carried out under the conditions of a catalyst holding temperature (T₂) of 280°C and a catalyst holding time of 6 h while flowing nitrogen at 1.9 NL/h.

### [Step 3: Isomerization Reaction Step]

The catalyst continuous reaction apparatus after Step 2 was subjected to a continuous reaction under the same conditions as in the above-described evaluation of catalytic activity. In the reaction solution collected at the end of the reaction, the amount of the product was determined by the above-described method, and the average double bond position (Ave.DBP₂) was calculated.

The larger the difference between Ave.DBP₂ and Ave.DBP₁ calculated above (ΔAve.DBP (= Ave.DBP₂ - Ave.DBP₁)) is, the higher the effect of regenerating the activity of the catalyst can be evaluated.

### Examples 2 to 11

Each step was performed in the same manner as in Example 1 except that the type and the liquid feeding amount of raw material olefin,, the charge amount of the catalyst used, the nitrogen flow rate, the reaction temperatures T₁ and T₃, the catalyst holding temperature T₂, and the reaction time were changed as shown in Table 1, and the amount of the product was determined in each step.

In Examples 10 and 11 in which 1-octadecene (manufactured by FUJIFILM Wako Pure Chemical Corporation) was used as the raw material olefin, the average double bond position (Ave.DBP) was calculated from the olefin content at each double bond position according to the following Formula 2. Ave.DBP = (1-olefin + 2 × 2-olefin + 3 × 3-olefin + 4 × 4-olefin + 5 × 5-olefin + 6 × 6-olefin + 7 × 7-olefin + 8 × 8-olefin + 9 × 9-olefin)/100

**Table 1**

| | | | Example 1 | Example 2 | Example 3 | Example 4 |
|---|---|---|---|---|---|---|
| Raw material olefin | | - | 1-Hexadecene | 1-Hexadecene | 1-Hexadecene | 1-Hexadecene |
| Catalyst | | - | γ-Alumina catalyst | γ-Alumina catalyst | γ-Alumina catalyst | γ-Alumina catalyst |
| Step 1 (Isomerization reaction step) | Charge amount of catalyst | mL | 400 | 400 | 400 | 400 |
| | Liquid feeding amount of raw material olefin | mL/hr | 100 | 100 | 100 | 100 |
| | N₂ flow rate | NL/hr | 3.9 | 3.9 | 3.9 | 3.9 |
| | N₂ pressure | MPaG | 0.1 | 0.1 | 0.1 | 0.1 |
| | Reaction Temperature T₁ | °C | 230 | 230 | 230 | 230 |
| | Reaction time | hr | 3,216 | 3,216 | 3,216 | 3,216 |
| Step 2 Catalyst regeneration step | Charge amount of catalyst | mL | 10 | 10 | 10 | 10 |
| | Liquid feeding amount of raw material olefin | mL/hr | 80 | 80 | 80 | 80 |
| | N₂ flow rate | NL/hr | 1.9 | 1.9 | 1.9 | 1.9 |
| | N₂ pressure | MPaG | 0 | 0 | 0 | 0 |
| | Catalyst holding temperature T₂ | °C | 280 | 260 | 300 | 300 |
| | Catalyst holding time | hr | 6 | 6 | 6 | 6 |
| Catalytic activity evaluation after Step 1 | Charge amount of catalyst | mL | 10 | 10 | 10 | 10 |
| | Liquid feeding amount of raw material olefin | mL/hr | 80 | 80 | 80 | 80 |
| or | N₂ flow rate | NL/hr | 1.9 | 1.9 | 1.9 | 1.9 |
| Catalytic activity evaluation after Step 2 (Step 3) | N₂ pressure | MPaG | 0 | 0 | 0 | 0 |
| | Reaction temperature T₃ | °C | 260 | 240 | 260 | 210 |
| | Reaction time | hr | 6 | 6 | 6 | 6 |
| Difference ΔT(T₂ - T₁) between T₂ in Step 2 and T₁ in Step 1 | | °C | 50 | 30 | 70 | 70 |
| Difference ΔT(T₂ - T₃) between T₂ in Step 2 and T₃ in Step 3 | | °C | 20 | 20 | 40 | 90 |
| Ave.DBP₁ | | - | 2.23 | 2.10 | 2.23 | 1.91 |
| Ave.DBP₂ | | - | 2.55 | 2.13 | 2.70 | 2.11 |
| ΔAve.DBP (= Ave.DBP₂ - Ave.DBP₁) | | - | 0.32 | 0.03 | 0.47 | 0.20 |

**Table 1 (continued)**

| | | | Example 5 | Example 6 | Example 7 | Example 8 |
|---|---|---|---|---|---|---|
| Raw material olefin | | - | 1-Hexadecene | 1-Hexadecene | 1-Hexadecene | 1-Hexadecene |
| Catalyst | | - | γ-Alumina catalyst | γ-Alumina catalyst | γ-Alumina catalyst | γ-Alumina catalyst |
| Step 1 (Isomerization reaction step) | Charge amount of catalyst | mL | 400 | 400 | 400 | 400 |
| | Liquid feeding amount of raw material olefin | mL/hr | 100 | 100 | 100 | 100 |
| | N₂ flow rate | NL/hr | 3.9 | 3.9 | 3.9 | 3.9 |
| | N₂ pressure | MPaG | 0.1 | 0.1 | 0.1 | 0.1 |
| | Reaction Temperature T₁ | °C | 230 | 230 | 230 | 230 |
| | Reaction time | hr | 3,216 | 3,216 | 3,216 | 3,216 |
| Step 2 Catalyst regeneration step | Charge amount of catalyst | mL | 10 | 10 | 10 | 10 |
| | Liquid feeding amount of raw material olefin | mL/hr | 80 | 40 | 40 | 20 |
| | N₂ flow rate | NL/hr | 1.9 | 1.9 | 1.9 | 1.9 |
| | N₂ pressure | MPaG | 0 | 0 | 0 | 0 |
| | Catalyst holding temperature T₂ | °C | 300 | 300 | 300 | 300 |
| | Catalyst holding time | hr | 6 | 6 | 12 | 6 |
| Catalytic activity evaluation after Step 1 | Charge amount of catalyst | mL | 10 | 10 | 10 | 10 |
| | Liquid feeding amount of raw material olefin | mL/hr | 80 | 80 | 80 | 80 |
| or | N₂ flow rate | NL/hr | 1.9 | 1.9 | 1.9 | 1.9 |
| Catalytic activity evaluation after Step 2 (Step 3) | N₂ pressure | MPaG | 0 | 0 | 0 | 0 |
| | Reaction temperature T₃ | °C | 180 | 260 | 260 | 260 |
| | Reaction time | hr | 6 | 6 | 6 | 6 |
| Difference ΔT(T₂ - T₁) between T₂ in Step 2 and T₁ in Step 1 | | °C | 70 | 70 | 70 | 70 |
| Difference ΔT(T₂ - T₃) between T₂ in Step 2 and T₃ in Step 3 | | °C | 120 | 40 | 40 | 40 |
| Ave.DBP₁ | | - | 1.76 | 2.21 | 2.21 | 2.23 |
| Ave.DBP₂ | | - | 1.88 | 2.50 | 2.63 | 2.35 |
| ΔAve.DBP(=Ave.DBP₂-Ave.DBP₁) | | - | 0.12 | 0.29 | 0.42 | 0.12 |

**Table 1 (continued)**

| | | | Example 9 | Example 10 | Example 11 |
|---|---|---|---|---|---|
| Raw material olefin | | - | 1-Hexadecene | 1-Octadecene | 1-Octadecene |
| Catalyst | | - | γ-Alumina catalyst | γ-Alumina catalyst | γ-Alumina catalyst |
| Step 1 (Isomerization reaction step) | Charge amount of catalyst | mL | 400 | 10 | 10 |
| | Liquid feeding amount of raw material olefin | mL/hr | 100 | 10 | 10 |
| | N₂ flow rate | NL/hr | 3.9 | 3.9 | 3.9 |
| | N₂ pressure | MPaG | 0.1 | 0.1 | 0.1 |
| | Reaction Temperature T₁ | °C | 230 | 255 | 255 |
| | Reaction time | hr | 3,216 | 1,526 | 1,526 |
| Step 2 Catalyst regeneration step | Charge amount of catalyst | mL | 10 | 10 | 10 |
| | Liquid feeding amount of raw material olefin | mL/hr | 20 | 10 | 10 |
| | N₂ flow rate | NL/hr | 1.9 | 1.9 | 1.9 |
| | N₂ pressure | MPaG | 0 | 0 | 0 |
| | Catalyst holding temperature T₂ | °C | 300 | 280 | 260 |
| | Catalyst holding time | hr | 24 | 12 | 12 |
| Catalytic activity evaluation after Step 1 | Charge amount of catalyst | mL | 10 | 10 | 10 |
| | Liquid feeding amount of raw material olefin | mL/hr | 80 | 80 | 10 |
| or | N₂ flow rate | NL/hr | 1.9 | 1.9 | 0.24 |
| Catalytic activity evaluation after Step 2 (Step 3) | N₂ pressure | MPaG | 0 | 0 | 0 |
| | Reaction temperature T₃ | °C | 260 | 260 | 240 |
| | Reaction time | hr | 6 | 6 | 6 |
| Difference ΔT(T₂ - T₁) between T₂ in Step 2 and T₁ in Step 1 | | °C | 70 | 25 | 5 |
| Difference ΔT(T₂ - T₃) between T₂ in Step 2 and T₃ in Step 3 | | °C | 40 | 20 | 20 |
| Ave.DBP₁ | | - | 2.23 | 2.25 | 2.73 |
| Ave.DBP₂ | | - | 2.59 | 2.30 | 2.76 |
| ΔAve.DBP(=Ave.DBP₂-Ave.DBP₁) | | - | 0.36 | 0.05 | 0.03 |

From the results of Examples of the present invention, it was confirmed that in the method for producing an internal olefin of the present invention, a catalyst having a decreased activity can be reactivated.

## Claims

1. A method for producing an internal olefin, including a step of subjecting a raw material olefin to an isomerization reaction in the presence of the following catalyst R:
Catalyst R: a catalyst obtained by holding a catalyst, which has been subjected to an isomerization reaction of an olefin at a reaction temperature (T₁), at a temperature higher than the reaction temperature (T₁) (hereinafter also referred to as a "catalyst holding temperature (T₂)") in the presence of the olefin.

2. The method for producing an internal olefin according to claim 1, wherein the catalyst R is a catalyst obtained by holding a catalyst, which has been subjected to an isomerization reaction of a raw material olefin at a reaction temperature (T₁), at a catalyst holding temperature (T₂) higher than the reaction temperature (T₁) in the presence of a raw material olefin.

3. The method for producing an internal olefin according to claim 1 or 2, wherein the raw material olefin has 12 or more and 24 or less carbon atoms.

4. The method for producing an internal olefin according to any one of claims 1 to 3, wherein the raw material olefin contains a 1-olefin.

5. The method for producing an internal olefin according to any one of claims 1 to 4, wherein a difference ΔT(T₂ - T₁) between the catalyst holding temperature (T₂) and the reaction temperature (T₁) is higher than 30°C and 150°C or lower.

6. The method for producing an internal olefin according to any one of claims 1 to 5, wherein the raw material olefin is subjected to an isomerization reaction at a reaction temperature (T₃) in the presence of the catalyst R, and the reaction temperature (T₃) is lower than the catalyst holding temperature (T₂).

7. The method for producing an internal olefin according to claim 6, wherein a difference ΔT(T₂ - T₃) between the catalyst holding temperature (T₂) and the reaction temperature (T₃) is 10°C or higher and 130°C or lower.

8. The method for producing an internal olefin according to any one of claims 1 to 7, wherein the reaction temperature (T₁) is 160°C or higher and 300°C or lower.

9. The method for producing an internal olefin according to any one of claims 1 to 8, wherein the catalyst is a solid Lewis acid catalyst.

10. The method for producing an internal olefin according to any one of claims 1 to 9, wherein the catalyst is an aluminum oxide catalyst.

11. A method for regenerating the activity of a catalyst, including the following step:
a step of holding the catalyst, which has been subjected to an isomerization reaction of an olefin at a reaction temperature (T₁), at a catalyst holding temperature (T₂) higher than the reaction temperature (T₁) in the presence of the olefin.

12. The method for regenerating the activity of a catalyst according to claim 11, wherein the step is a step of holding a catalyst, which has been subjected to an isomerization reaction of a raw material olefin at a reaction temperature (T₁), at a catalyst holding temperature (T₂) higher than the reaction temperature (T₁) in the presence of a raw material olefin.

13. The method for regenerating the activity of a catalyst according to claim 12, wherein the raw material olefin has 12 or more and 24 or less carbon atoms.

14. The method for regenerating the activity of a catalyst according to claim 12 or 13, wherein the raw material olefin contains a 1-olefin.

15. The method for regenerating the activity of a catalyst according to any one of claims 11 to 14, wherein the catalyst is a solid Lewis acid catalyst.

16. The method for regenerating the activity of a catalyst according to any one of claims 11 to 15, wherein the catalyst is an aluminum oxide catalyst.
